# EUROPEAN PATENT APPLICATION

(11) **EP 2 008 566 A1**
(43) Date of publication of application: **31.12.2008**
(21) Application number: 08156331.4
(22) Date of filing: 16.05.2008
(51) Int. Cl.: A47L 13/17

(54) **Liquid delivery pouch and article comprising the same**

(30) Priority: 25.06.2007 US 937032 P; 22.08.2007 US 957244 P
(71) Applicant: The Procter and Gamble Company, Cincinnati, Ohio 45202 (US)
(72) Inventor: Pung, David, John, Loveland, OH 45140 (US); Mortland, Kim, Morey, Cincinnati, OH 45242 (US); Thuemmler, Karl, Edward, Liberty Township, OH 45011 (US)
(74) Representative: Morelle, Evelyne Charlotte Isabelle

(57) **Abstract**

A liquid delivery pouch (10) comprises an outer liquid-impermeable wall (20) and a liquid contained therein. The liquid-impermeable wall (20) comprises at least one aperture (40) having a diameter of from 200 µm to 500 µm. The at least one aperture (40) is sealed by a sealing means (60) to prevent the liquid to be released from said pouch (10) prior to use.

## Description

### FIELD OF THE INVENTION

The present invention relates to a liquid delivery pouch, to an article comprising said pouch and a substrate, and to a method for the controlled delivery of a liquid.

### BACKGROUND OF THE INVENTION

Wet wipes are known in the art. They are used in various fields to deliver a liquid to a surface. For example, wet wipes are used as cleaning and polishing wipes for household surfaces, or as cleansing or skin care wipes. However, wet wipes suffer from a number of disadvantages.
Wet wipes often drip when they are removed from the package, especially as you reach the end of the pack. Frequently, the user's hands come into contact with the liquid impregnated into the wipe. This makes wet wipes difficult to handle, which is perceived as a negative by users.
Another problem which often occurs is that the wet wipes have dried out in the package during storage, especially if left in storage over extended period of time or if the package is left open. Moreover, many wet wipes do not allow for effective delivery of the impregnated liquid to a surface. Often, too much liquid is released in the beginning, leaving little liquid left to treat large surface areas.

Pouches are described in the prior art to overcome the above problems. By enclosing the liquid in an impermeable pouch, drying out and dripping is prevented and the user's hand do not come in direct contact with the liquid. The dimensions of the pouch, and the amount of liquid stored in the pouch can be adjusted to provide the required mileage such that larger surface areas can be treated.

In order to release the liquid from the pouch, a number of solutions are known. One such solution is a rupturable pouch comprising a frangible seal, When pressure is applied to the pouch, the pouch bursts and the liquid is released. Rupturable pouches however have the disadvantage that it is difficult to control the size of the opening created after rupturing. As a result, an overly large opening may be formed, thereby releasing most of the liquid at once. Also rupturable pouches may burst unintentionally during storage and transport.

Another example, as described in US 2006/0096051 (Kao Corporation) is a pouch with preformed, elongated openings which are closed by a removable seal. At the time of use, a consumer removes the seal to enable the liquid to flow out from the pouch. In order to prevent liquid from flowing out all at once when the seal is removed, the liquid is absorbed in an absorbent substrate inside the pouch. While this execution allows more control over the liquid release, it requires more pressure to be exerted by a user. Moreover, such an execution is not cost efficient.

Accordingly, it is an objective of the present invention to overcome the problems of the prior art, and to provide a pouch and article comprising said pouch with better controlled delivery of the liquid to a target surface.

### SUMMARY OF THE INVENTION

According to a first aspect, the present invention relates to a pouch comprising an outer liquid-impermeable wall and a liquid contained within said pouch. The liquid-impermeable wall comprises at least one aperture having a diameter of from 200 µm to 500 µm. The at least one aperture is sealed by a sealing means to prevent the liquid from being released from said pouch prior to use.

According to a second aspect, the present invention relates to the pouch in combination with a substrate.

In a third aspect, the present invention relates to a method for the controlled release of a liquid, comprising the steps of;
a. providing a pouch as described above; then
b. removing the sealing means to expose the at least one aperture; then
c. releasing the liquid from the pouch by means of gravity, pressure, or combinations thereof.

### BRIEF DESCRIPTION OF THE DRAWINGS

Fig. 1 shows a side view of a pouch according to the present invention.
Fig. 2 shows an underneath view of an embodiment of a pouch according to the present invention.
Fig. 3 shows a cross-section of another embodiment of a pouch according to the present invention.
Fig. 4 shows a side view of another embodiment of a pouch according to the present invention.
Fig. 5 shows an underneath view of another embodiment of a pouch according to the present invention.
Fig. 6 shows an underneath view of another embodiment of a pouch according to the present invention.
Fig. 7 shows a side view of another embodiment of a pouch according to the present invention.
Fig. 8 shows a side view of another embodiment of a pouch according to the present invention.
Fig. 9 shows an upper view of an article comprising a pouch according to the present invention.

### DETAILED DESCRIPTION OF THE INVENTION

The present invention relates to a pouch for the controlled delivery of one or more liquids to a target surface.

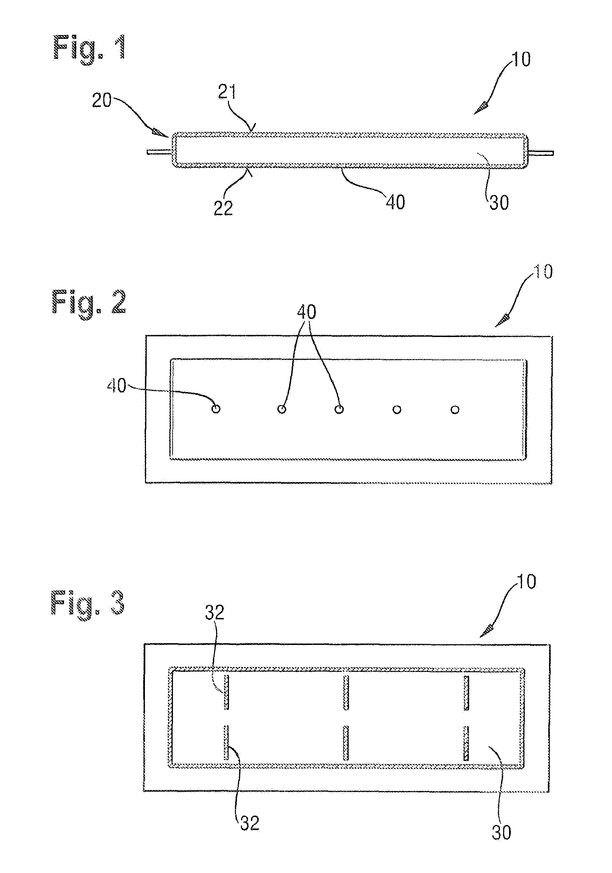

As shown in Fig. 1, the pouch **10** is a flexible film reservoir comprising an outer liquid-impermeable wall **20** defining a cavity **30** for holding one or more liquids. Typically the pouch has an upper side **21** and a lower side **22,** said upper and lower sides **21, 22** being sealed along the perimeter. The liquid-impermeable wall **20** forms a barrier to the liquid such that the pouch is capable of safely storing liquids under various environmental, transporting and storage conditions.

The liquid-impermeable wall can be a mono-layer or a multi-layer structure. The barrier properties of the mono-layer structure may be further enhanced by an additional barrier-coating which for example can be cold- or heat-sealed to the mono-layer structure. The multi-layer structure may for example be a laminate structure comprising a barrier layer and a sealable layer. The barrier layer is liquid-impermeable and provides the necessary barrier properties to prevent the liquid from escaping from the pouch **10.** The sealable layer forms an inner layer and allows the laminate to be sealed at the perimeter in order to form a pouch, and is preferably a heat-sealable layer. The sealable layer is preferably also impervious to the liquid stored in the pouch. Optionally, the laminate structure may further comprise an outer layer which is preferably printable to enhance the overall appearance and aesthetics of the pouch.

Suitable liquid-impermeable materials for a single-layer structure include, but are not limited to, bi-oriented polypropylene, metallised polypropylene, or barrier-coated polyethylene.
Suitable materials for the barrier layer of a laminate structure include, but are not limited thereto, bi-oriented polypropylene, metallised polypropylene, metallocene polyethylene, metallic foil, and modified Nylon™.
Suitable materials for the sealable layer of a laminate structure include, but are not limited thereto, linear low density polyethylene (LLDPE), low density polyethylene (LDPE), high density polyethylene (HDPE), ethylene vinyl acetate (EVA), ethylene copolymers like Surlyn™.

As shown in Fig. 2, the liquid-impermeable wall **20** comprises at least one aperture **40** having an average diameter of from 200 µm to 500 µm. Preferably the at least one aperture **40** has an average diameter of from 250 µm to 450 µm, more preferably from 300 µm to 400 µm. While gravitational forces would allow the liquid to pass through the aperture, the diameter ranges of the present invention provide the user with better control for releasing the liquid from the pouch. It has been surprisingly found that these diameter ranges provide the best release properties under pressures typically exerted by a user on wet wipes. If the aperture would be smaller, too much pressure would be required to efficiently release the liquid from the pouch. If the aperture would be larger, the user would have no control over the release, as the liquid would flow immediately. The aperture can be created by any suitable method, including mechanical needle aperturing, hot needle aperturing, laser etching, and vacuum forming. Alternatively, a microporous liquid-impermeable film with pores having a diameter as specified above, may be used.
The number of apertures can be varied depending on the dimensions of the pouch and the intended use. It is preferred that the apertures are arranged on one side of the pouch only.
Preferably the pouch **10** comprises an array of apertures **40.** The apertures **40** can be arranged evenly over the surface of the pouch, or they can be grouped at one or more locations where more pressure is typically exerted by a user.

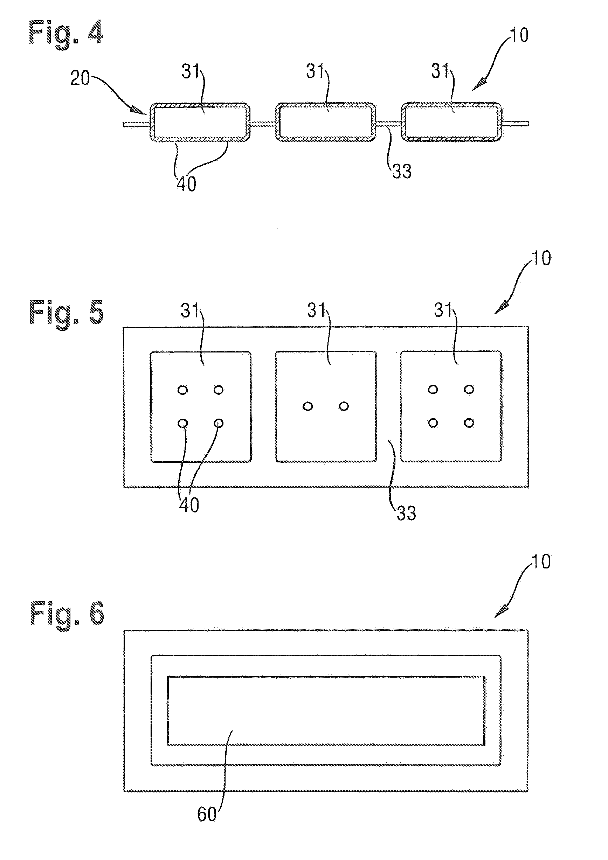

The cavity **30** can be a single compartment. Flow restriction means **32** may be incorporated inside the pouch, as shown in Fig. 3. Alternatively, as shown in Fig. 4, the pouch **10** may comprise **2** or more compartments **31.** The compartments **31** are separated by a liquid-impermeable seal **33.** Each compartment **31** comprises a liquid. Each compartment may comprise the same liquid and each compartment **31** comprises at least one aperture **40.** Each compartment **31** may comprise the same number of apertures **40,** or they may comprise a different number of apertures **40.** The specific execution depends on the dimensions of the pouch 10 and where pressure is to be applied. When pressure is evenly applied over the entire width of the pouch **10,** it is preferred to have an equal number of apertures **40** in each compartment **31.** When pressure is applied mainly at one or more locations of the pouch **10,** it is preferred to have a different number of apertures **40** in the compartment(s) **31** at these pressure locations compared to the other compartments **31** present where less pressure is exerted, as shown in Fig 5.
In another embodiment, each compartment comprises a different liquid. The liquid-impermeable seal **33** may be a frangible seal such that, under pressure, the seal breaks and the liquids mix before they flow out the pouch. Alternatively, the liquids may mix on the surface where they are applied to.

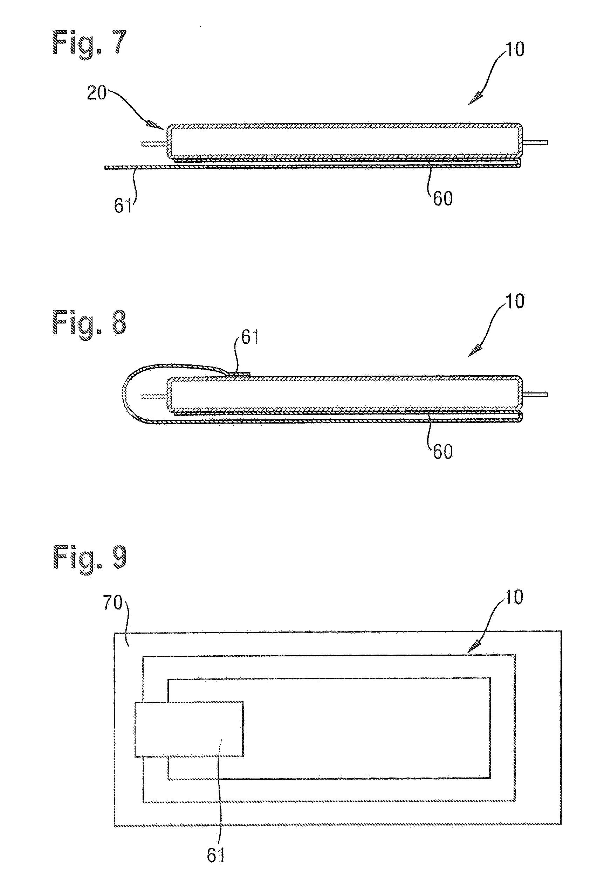

In order to prevent the liquid from flowing out prematurely of the pouch **10,** the at least one aperture **40** is sealed by a sealing means **60** as shown in Fig. 6. Prior to use, the sealing means **60** is removed from the pouch **10** to expose the at least one aperture **40.** The sealing means **60** is also liquid-impermeable. The sealing means **60** can be attached to the pouch **10** for example by a light-tack hermetic heat seal or by light-tack pressure-sensitive adhesive seal. Suitable materials for a light-tack hermetic seal include, but are not limited thereto, Surlyn™, oriented polypropylene, bi-oriented polypropylene, polyethene terephtalate, low density polyethylene, linear low density polyethylene with a coating of temperature- or adhesive-activated adhesive, and mixtures thereof. The adhesive can be solvent- or water-based and is selected to be compatible with the liquid stored inside the pouch, be capable of withstanding long-term contact with the liquid to ensure the pouch is leaktight, and be able to withstand varying environmental temperatures, pressures and humidity conditions.
Preferably, the sealing means **60** comprises a pull tab **61.** Preferably, the pull tab **61** extends beyond the dimensions of the pouch **10,** as shown in Fig. 7. This allows a user to easily remove the sealing means **60** prior to use.
The pull tab **61** may optionally be attached back to the pouch, preferably to the side opposite to the side comprising the sealing means 60, by means of a low-tack adhesive applied at the end of the tab. This allows for easier converting and packaging of the pouch. The pull tab can be easily released by a user as a loop is created underneath which the user can insert his finger, as shown in Fig. 8.

The liquid contained within the pouch **10** can be any suitable liquid which can be applied to a surface. For example it can be a household cleaning or polishing liquid or it can be a liquid for the cleansing or caring of human skin or hair.
In one embodiment, the liquid is an aqueous, household cleaning composition. The composition preferably comprises one or more surfactants. The surfactants are present at a level_from about 0.005% to about 0.50% surfactants. Suitable surfactants include nonionic, zwitterionic, amphoteric, anionic or cationic surfactants, having hydrophobic chains containing from about 8 to about 18 carbon atoms. Examples of suitable surfactants are described in McCutcheon's Vol. 1: Emulsifiers and Detergents, North American Ed., McCutcheon Division, MC Publishing Co., 2002. Preferably, the aqueous compositions comprise from about 0.005% to about 0.45%, more preferably from about 0.0075% to about 0.30%, still more preferably from about 0.01% to about 0.20%, and most preferably from about 0.015% to about 0.10% surfactants.
The composition may optionally comprise one or more solvents. Solvents lower surface tension properties of the composition thereby helping wetting and cleaning of floor surfaces. Optional solvents for use herein include all those known in the art for use in hard-surface cleaner compositions. Suitable solvents can be selected from the group consisting of: aliphatic alcohols, ethers and diethers, glycols or alkoxylated glycols, glycol ethers, alkoxylated aromatic alcohols; aromatic alcohols, terpenes, and mixtures thereof. Aliphatic diols and glycol ether solvents are most preferred solvents. If present, solvents are preferably present at levels from about 0.25% to about 10%, more preferably about 0.5% to about 5%, more preferably from about 1% to about 4% by weight of the aqueous cleaning compositions.
The following polymers are highly preferred optional ingredients that can offer additional benefits, including but not limited to, viscosity modification, haze mitigation, gloss enhancement and particulate soil removal. Of particular interest are the specific polymers or classes of polymers disclosed in European Patent Application No. 1 019 475, European Patent Application 1 216 295, U.S. Patent No. 6,340,663, U.S. Patent Application No. 2003/0017960, U.S. Patent Application No. 2003/0186830; and WO 01/23510. Non-limiting examples of suitable polymers include naturally occurring polysaccharides such as xanthan gum, guar gum, locust bean gum and synthetic polysaccharides such carboxymethylcellulose, ethyl cellulose, hydroxyethyl cellulose, hydroxypropyl cellulose. Other suitable polymers include those derived from N-vinyl pyrrolidone, including polyvinyl pyrrolidones (10,000 to 200,000 molecular weight) and copolymers formed by reacting N-vinyl pyrrolidone with either acrylic acid, methacrylic acid, itaconic acid, caprolactam, butene or vinyl acetate. Still other suitable polymers comprise sulfonate and amine oxide functionalities, such as polyvinyl pyridine-N-oxide (1,000 to 50,000 molecular weight), polyvinyl sulfonate (1,000 to 10,000 molecular weight), and polyvinyl styrene sulfonate (10,000 to 1,000,000 molecular weight). Yet other classes of suitable polymers include polyethylene glycols (5,000 to 5,000,000 molecular weight), modified polyethylene imines such as Lupasol SK sold by BASF (100,000 to 5,000,000 molecular weight). Other classes of suitable polymers include acrylate-based or substituted acrylate-based polymers or copolymers. Examples of acrylates and substituted acrylates include sodium acrylate, sodium methacrylate, potassium ethyl acrylate and potassium butyl methacrylate. Most preferred are sodium acrylate and sodium methacrylate.
The composition may comprise a variety of other optional ingredients depending on the technical benefit aimed for and the surface treated. Suitable optional ingredients for use herein include additional chelants, builders, enzymes buffers, perfumes, hydrotropes, colorants, pigments and/or dyes. In most cases, it is preferable that the level of these components not exceed about 0,50% of the composition.

When the pouch **10** comprises several compartments, each compartment may comprise a different liquid, including liquids which are incompatible with each other and which only come into contact with each other at the point of use or aggressive liquids such as for example bleach.

The liquid suitable for use in the present invention may cover a broad range of viscosities, so long as the liquid either readily flows or can otherwise be dispensed or discharged from the pouch by pressure applied thereto.

The pouch **10** of the present invention is preferably used in combination with a substrate **70.** The substrate **70** improves the application of the liquid to the target surface. The substrate **70** is selected based on the end use of the product and on the desired liquid release. The substrate **70** may be comprised of a woven substrate, a nonwoven substrate, a foam, a sponge or combinations thereof. Preferably, the substrate **70** comprises a nonwoven substrate. The nonwoven substrate may be made by, but not limited to, any of the following methods: spunlaced, spunbond, meltdown, carded thermal bonded, hydroapertured, hydroentangled, carded, air through bonded, calendar bonded, or combinations thereof. Carded thermal bonding and hydroentangling are preferred.

The term "nonwoven" as used herein is a term recognized in the art which distinguishes over woven fabrics in that the nonwoven substrate contain fibers that are not woven into a fabric but rather are formed into a sheet, mat, or pad layer. The fibers within the nonwoven substrate can be randomly aligned, oriented in primarily one direction, or otherwise oriented in some other nonwoven pattern depending upon the fluid entanglement manufacturing process used.

The nonwoven substrate may be derived from or comprised of a variety of materials both natural and synthetic. By natural is meant that the materials are derived from plants, animals, insects or byproducts of plants, animals, and insects. By synthetic is meant that the materials are obtained primarily from various man-made materials or from natural materials which have been further altered. The conventional base starting material is usually a fibrous web comprising any of the common synthetic or natural textile-length fibers, or mixtures thereof. Nonlimiting examples of natural materials may include silk fibers, keratin fibers, cellulosic fibers and combinations thereof. Nonlimiting examples of keratin fibers include wool fibers, camel hair fibers, and other similar materials. Nonlimiting examples of cellulosic fibers include wood pulp fibers, cotton fibers, hemp fibers, jute fibers, flax fibers, and combinations thereof. Nonlimiting examples of synthetic materials may include those acetate fibers, acrylic fibers, cellulose ester fibers, modacrylic fibers, polyamide fibers, polyester fibers, polyolefin fibers, polyvinyl alcohol fibers, rayon fibers, polyurethane foam, and combinations thereof. Nonlimiting examples of synthetic materials include acrylics such as acrilan, crcslan, and the acrylonitrile-based fiber (orlon); cellulose ester fibers such as cellulose acetate, arnel, and acele; polyamides such as nylons (e.g., nylon 6, nylon 66, nylon 610, and the like); polyesters such as fortrel, kodel, and the polyethylene terephthalate fiber, dacron; polyolefins such as polypropylene, polyethylene; polyvinyl acetate fibers; polyurethane foams and mixtures thereof.

The fibers useful herein can be hydrophilic, hydrophobic, or can be a combination of both hydrophilic and hydrophobic fibers. Suitable hydrophilic fibers include cellulosic fibers, modified cellulosic fibers, rayon, cotton, polyester fibers such as hydrophilic nylon (HYDROFIL®). Suitable hydrophilic fibers can also be obtained by hydrophilizing hydrophobic fibers, such as surfactant-treated or silica-treated thermoplastic fibers derived from, for example, polyolefins such as polyethylene, polypropylene, polyacrylics, polyamides, polystyrenes, polyurethanes and the like. Suitable wood pulp fibers can be obtained from well-known chemical processes such as the Kraft and sulfite processes. It is especially preferred to derive these wood pulp fibers from southern soft woods due to their premium absorbency characteristics. These wood pulp fibers can also be obtained from mechanical processes, such as ground wood, refiner mechanical, thermomechanical, chemimechanical, and chemi-thermomechanical pulp processes. Recycled or secondary wood pulp fibers, as well as bleached and unbleached wood pulp fibers, can be used. Another type of hydrophilic fibers for use in the present invention are chemically stiffened cellulosic fibers. As used herein, the term "chemically stiffened cellulosic fibers" means cellulosic fibers that have been stiffened by chemical means to increase the stiffness of the fibers under both dry and aqueous conditions. Such means can include the addition of a chemical stiffening agent that, for example, coats and/or impregnates the fibers. Such means can also include the stiffening of the fibers by altering the chemical structure, e.g., by crosslinking polymer chains.

Thermoplastic materials useful in the present invention can be in any of a variety of forms including particulates, fibers, or combinations of particulates and fibers. Thermoplastic fibers are a particularly preferred form because of their ability to form numerous interfiber bond sites. Suitable thermoplastic materials can be made from any thermoplastic polymer that can be melted at temperatures that will not extensively damage the fibers that comprise the primary substrate or matrix of each layer. Preferably, the melting point of this thermoplastic material will be less than about 190°C, and preferably between about 75°C and about 175°C. In any event, the melting point of this thermoplastic material should be no lower than the temperature at which the thermally bonded absorbent structures, when used in the cleaning pads, are likely to be stored. The melting point of the thermoplastic material is typically no lower than about 50°C.
The thermoplastic materials, and in particular the thermoplastic fibers, can be made from a variety of thermoplastic polymers, including polyc-ylefins such as polyethylene (e.g., PULPEX®) and polypropylene, polyesters, copolyesters, polyvinyl acetate, polyethylvinyl acetate, polyvinyl chloride, polyvinylidene chloride, polyacrylics, polyamides, copolyamides, polystyrenes, polyurethanes and copolymers of any of the foregoing such as vinyl chloride/vinyl acetate, and the like. Depending upon the desired characteristics for the resulting thermally bonded nonwoven substrate, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, polyurethanes and the like. The surface of the hydrophobic thermoplastic fiber can be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij® 76 manufactured by ICI Americas, Inc. of Wilmington, Delaware, and various surfactants sold under the Pegosperse® trademark by Glyco Chemical, Inc. of Greenwich, Connecticut. Besides nonionic surfactants, anionic surfactants can also be used. These surfactants can be applied to the thermoplastic fibers at levels of, for example, from about 0.2 to about 1 gram per square centimeter of thermoplastic fiber.
Suitable thermoplastic fibers can be made from a single polymer (monocomponent fibers), or can be made from more than one polymer (e.g., bicomponent fibers). As used herein, "bicomponent fibers" refers to thermoplastic fibers that comprise a core fiber made from one polymer that is encased within a thermoplastic sheath made from a different polymer. The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.
Suitable bicomponent fibers include sheath/core fibers having the following polymer combinations: polyethylene/ polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., those available from Danaklon a/s, Chisso Corp., and CELBOND®, available from Hercules). These bicomponent fibers can be concentric or eccentric. As used herein, the terms "concentric" and "eccentric" refer to whether the sheath has a thickness that is even, or uneven, through the cross-sectional area of the bicomponent fiber. Eccentric bicomponent fibers can be desirable in providing more compressive strength at lower fiber thicknesses.
Methods for preparing thermally bonded fibrous materials are described in U.S. application Serial No. 08/479,096 (Richards et al.), filed July 3, 1995 (see especially pages 16-20) and U.S. Patent 5,549,589 (Homey et al.), issued August 27, 1996 (see especially Columns 9 to 10).

The nonwoven fabric has a basis weight of preferably from 50 gsm to 100 gsm, more preferably from 60 gsm to 80 gsm. Preferbaly the weight ratio of natural fibers (preferably cellulosic fibers) to synthetic fibers is 50:50, more preferably 60:80.
In one preferred embodiment, the nonwoven substrate comprises a hydroentangled nonwoven substrate. Hydroentangled nonwoven substrates and the fluid entangling techniques for making them are well known in the substrate art, preferred examples of such substrates and fluid entangling techniques being described in U.S. Patents 5,142,752 (Greenway et al.) and U.S. Patents 5,281,461 (Greenway et al.). Other known techniques for making hydroentangled nonwoven substrates are described, for example in U.S. Patent 3,485,786 (Evans); U.S. Patent 2,862,251 (Kalwarres): and U.S. Patent 3,025,585 (Griswald). Other suitable methods of making hydroentangled substrates are described in U.S. Patent 5,674,591 (James et al.) which specifically describes a hydroentangling process, including the apparatus used in said process, which can be used to prepare a patterned web. The term "hydroentangled" as used herein is an art recognized term which refers generally to the manufacturing process for entangling a fibrous web by using a fluid jet on a fibrous web to obtain the desired fiber and void configuration within the resulting fluid entangled substrate, to thereby produce an art recognized, hydroentangled, nonwoven substrate.

The pouch **10** can be placed with the at least one aperture **40** facing the substrate **70** prior to use, and preferably, is then attached to the substrate **70.** Preferably, the pouch **10** is already attached to the substrate **70,** as shown in Fig. 8. The pouch 10 can be attached along its periphery, or it can be attached along one side, preferably a longitudinal side. The latter provides the convenience that the pouch 10 can be flipped away from the substrate, thereby exposing the sealing means **60** such that a user can easily remove the sealing means 60. When the sealing means **60** is removed, the pouch can be flipped back onto the substrate. The pull tab **61** of the sealing means **60** preferably extends beyond the dimensions of the susbtrate **70.**

The present invention also relates to a method for the controlled release of a liquid. The method comprises the steps of:
- providing a pouch as previously described; then
- removing the sealing means to expose the at least one aperture; then
- releasing the liquid from the pouch by means of gravity, pressure, or combinations thereof.

The method may further comprise the step of placing or attaching said pouch onto a substrate with the at least one aperture facing the substrate. Preferably, the sealing means is removed after the pouch is placed onto, or attached to the substrate.

For some applications, such as floor cleaning, the substrate with the pouch attached thereto, may be attached to a cleaning implement. A suitable cleaning implement comprises a handle and a cleaning head onto which the substrate can be attached. The substrate is attached such that the pouch is facing the cleaning head. The use of an implement in combination with the pouch-substrate combination may allow a user to provide more pressure or to better control the pressure applied to the pouch.

To improve the overall appearance and aesthetics of the pouch, the pouch may be colored or printed with graphics or branding. Alternatively, or in addition, the pouch may be printed with usage instructions.

### Example;

A pouch is made of a tri-layer laminate comprising a polyethyleneterephtalate/polyethylene outer layer, a metallised polyethyleneterephtalate/polyethylene middle layer and a sealable layer as inner layer supplied by Print-Pak Inc. (Atlanta, U.S.A.) or Alcan Industries (Neenah, U.S.A.), sealed to a dimension of about 10 cm wide to about 25 cm long.
3 equidistant seals are created to form 4 seperated fluid compartments. The pouch is filled with 80 grams of a cleaning composition, for example a Swiffer Wet™ solution at 20 grams per compartment.
2 holes of 350 µm, spaced apart about 3 cm, are created by laser in each compartment.
An adhesive label, with a pull tab attached to it, is applied over the holes with a pull tab attached. The pouch is adhesively attached along its edges to a 70 gsm carded thermal bonded substrate from Fiberweb to form a cleaning article.
The cleaning article is attached to the support head of a Swiffer™ implement, with the pouch facing the head.
The adhesive label is removed by pulling the pull tab.
A floor surface of about 16 m² is cleaned by moving the support head with the cleaning article attached thereto, over the surface.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A pouch comprising an outer liquid-impermeable wall and a liquid contained within said pouch, said liquid-impermeable wall comprising at least one aperture having a diameter of from 200 µm to 500 µm, said at least one aperture being sealed by a sealing means to prevent the liquid to be released from said pouch prior to use.

2. A pouch according to claim 1, wherein said aperture has an average diameter of from 250 µm to 350 µm.

3. A pouch according to any preceding claim, wherein said pouch comprises flow restriction means.

4. A pouch according to any preceding claim, wherein said pouch comprises at least 2 compartments separated from each other by a liquid-impermeable wall, each compartment having a liquid contained therein, and each compartment comprises at least one aperture sealed by said sealing means.

5. A pouch according to claim 4, wherein said at least 2 compartments comprise a different liquid.

6. A pouch according to claims 4-5, wherein said at least 2 compartments comprise a different number of apertures.

7. A pouch according to any preceding claim, wherein said sealing means comprises a pull tab

8. An article comprising:
a. a pouch according to claims 1-7, and
b. a substrate.

9. An article according to claim 8, wherein said substrate is a woven substrate, a nonwoven substrate, a foam substrate, a sponge, or combinations thereof.

10. An article according to claims 8-9, wherein said pouch is attached to said substrate, and wherein said at least one sealed aperture is facing said substrate.

11. An article according to claims 8-10, wherein said pull tab extends beyond the dimensions of said substrate.

12. A method for the controlled release of a liquid, comprising the steps of:
a. providing a pouch according to claims 1-7; then
b. removing said sealing means to expose said at least one aperture; then
c. releasing said liquid from said pouch by means of gravity, pressure, or combinations thereof.

13. A method according to claim 12, further comprising the step of attaching said pouch to a substrate such that said at least one aperture is facing said substrate, prior to removing said sealing means.

14. A method according to claim 12, wherein said pouch is attached to a substrate such that said at least one aperture is facing said substrate.

15. A method according to claims 13-14, wherein said substrate having said pouch attached thereto, is further attached to an implement or a device, prior to removing said sealing means.
